# EUROPÄISCHE PATENTSCHRIFT

(11) **EP 2 670 906 B1**
(45) Veröffentlichungstag und Bekanntmachung des Hinweises auf die Patenterteilung: **29.08.2018**
(21) Anmeldenummer: 12716199.0
(22) Anmeldetag: 02.02.2012
(51) Int. Cl.: D06M 15/333, D06M 15/356

(54) **VERFAHREN ZUR OBERFLÄCHENMODIFIZIERUNG VON AUS NIEDER-ENERGETISCHEN CHEMIEFASERN HERGESTELLTEN PRODUKTEN**
METHOD FOR THE SURFACE MODIFICATION OF PRODUCTS MADE OF LOW-ENERGY SYNTHETIC FIBRES
PROCÉDÉ DE MODIFICATION DE LA SURFACE DE PRODUITS EN FIBRES CHIMIQUES À BASSE ÉNERGIE

(30) Priorität: 03.02.2011 DE 102011010136
(43) Veröffentlichungstag der Anmeldung: 11.12.2013
(73) Patentinhaber: LMD Labor für molekulares Design GmbH, 97076 Würzburg (DE)
(72) Erfinder: SIEGEL, Rolf, 97076 Würzburg (DE)
(74) Vertreter: Prinz & Partner mbB
(86) Internationale Anmeldenummer: PCT/DE2012/000089
(87) Internationale Veröffentlichungsnummer: WO 2012/103876

(56) Entgegenhaltungen:
- DE-T2- 69 716 773
- JP-A- 2002 029 151

## Beschreibung

Die Erfindung betrifft ein Verfahren zur Oberflächenmodifizierung von aus nieder-energetischen Chemiefasern hergestellten Produkten, die Verfahrensprodukte und die Verwendung der Verfahrensprodukte.

Unter dem Begriff "Chemiefasern", wie er hier verwendet wird, werden im folgenden lineare, in Fadenform vorliegende Makromoleküle, die zum Beispiel den Stoffklassen der Aramide, Polyester, Polyamide, Polyacrylate, Polyacrylnitrile, Polyurethane, (per) fluorierte Polyolefine, z.B. Polytetrafluorethylen oder Polyvinyliden(di)fluorid, Polysulfone, Polyimide, Polyolefine etc. sowie Co- oder Terpolymeren davon, angehören können, verstanden.

Unter der Bezeichnung "aus Chemiefasern hergestellte Produkte" werden im folgenden geformte Artikel verstanden, die in 1- und/oder 2-dimensionaler Ausbildung vorliegen. In vorwiegend 1-dimensionaler Ausbildung sind dies z.B. Filamente, Mono-filamente, Multifilamente, Fasern, Fäden, Garne, Zwirne, Rovings etc.. Ihr Durchmesser kann sowohl im unteren µm-Bereich als auch im unteren mm-Bereich liegen, z.B. bei 6 µm einerseits und 3,4 mm andererseits. Die Länge kann im mm-Bereich (Stapelfasern) als auch im km-Bereich (Filamente) liegen. Der Querschnitt der Fasern kann rund, elliptisch, drei- oder viereckig, polygonal, sternförmig, lobulär, zylindrisch odgl. sein. Die unmodifizierte Faseroberfläche kann glatt oder rau sein. In vorwiegend 2-dimensionaler Ausbildung sind dies textile Fächengebilde, die als Fadenverbundstoff wie Gewebe, Gewirke, Fleece, Gestricke, Netze, Geflechte etc. oder als Faserverbundstoff wie Vliese, Filze, Watte etc. oder als Kombinationen aus Faden- und Faserverbundstoff wie Flächenschichtstoffe vorliegen. Das Flächengewicht ist nur durch die technische Machbarkeit limitiert und kann z.B. zwischen 6 g/m² einerseits und 1.600 g/m² andererseits liegen. - Im weiteren wird, insbesondere im Zusammenhang mit Oberflächenmodifkation(en), auch der Begriff "Material" für o.g. aus Chemiefasern hergestellte Produkte verwendet.

Der hier verwendete Begriff "Oberflächenmodifizierung" bedeutet, dass sich die Oberflächen von nicht-oberflächen-modifizierten Materialien von oberflächen-modifizierten Materialien in wenigstens einem der folgenden Punkte unterscheiden:
- in den Ergebnissen der Oberflächenanalytik, z.B. mittels ESCA (Electron Spectroscopy for Chemical Analysis) oder SIMS (Secondary Ion Mass Spectroscopy)
- in der Wasserbenetzbarkeit
- in der Elektrostatik
- in der chemischen Reaktivität, z.B. in der Färbbarkeit mit wasserlöslichen Farbstoffen.

Der Begriff "nieder-energetisch", wie er hier verwendet wird, bedeutet, dass aus nieder-energetischen Chemiefasern hergestellte Produkte wenigstens eines der folgenden Merkmale aufweisen:
- die freie Oberflächenenergie liegt unter 40 mN/m
- der Kontaktwinkel von Wasser ist größer als 45°, d.h. die Wasserbenetzbarkeit ist gering oder fehlt, das Material ist hydrophob.

Aus nieder-energetischen Chemiefasern hergestellte Produkte, insbesondere solche aus Polyolefinen, weisen eine Reihe von Nachteilen auf:
- die Materialoberflächen sind chemisch inert, sie weisen keine oder nur mit (chemisch) drastischen Mitteln aktivierbare funktionellen Gruppen auf, somit ist beispielsweise eine Färbung direkt aus wässriger Phase heraus nicht möglich.
- die Materialien laden sich leicht elektrostatisch auf, wodurch ein weitergehender Einsatz im technischen Bereich verhindert wird.
- die Materialien sind nicht wasserbenetzbar. Eine textile Verwendung, z.B als Futterstoff, verbietet sich, da man aufgrund der fehlenden Schweißabsorption sofort schwitzen würde. Ein Einsatz als Wundauflage / Hygieneprodukt scheitert am fehlenden Absorptions- bzw. Weiterleitungsvermögen für Wundsekret / Körperflüssigkeit. Eine Verwendung als Filtermaterial für wässrige Medien ist nicht möglich, da das Wasser das Material nicht durchdringt.

Lösungen, die zur Behebung o.g. Nachteile vorgeschlagen wurden, umfassen entweder die Ausrüstung der Oberfläche von nieder-energetischen Materialien mit oberflächenaktiven Substanzen, wie Netzmittel und Tenside oder aber die Abscheidung hydrophiler Polymere wie Polyvinylpyrrolidon oder Polyvinylalkohol auf der Oberfläche. Die vorgeschlagenen Lösungen befriedigen nicht, da die Ausrüstung mit oberflächenaktiven Substanzen nicht dauerhaft ist, sie werden bei Kontakt mit Wasser ab- / ausgewaschen. Hydrophile Polymere müssen, damit sie auf der Oberfläche halten, mit zum Teil toxischen Substanzen vernetzt werden, dies ist aufwendig und kostspielig.

Aus der JP-A 2002-029151 ist ein Verfahren zur Herstellung von Kunstpapier bekannt, bei dem die Oberfläche eines aus Polyesterfasern gebildeten Papiers mit einer wässrigen Suspension von 100 Teilen Siliziumoxid, 30 Teilen eines silylierten Polyvinylalkohols und 5 Teilen eines kationischen Polymers behandelt wird.

Die EP 1 000 188 B1 beschreibt einen absorbierenden Gegenstand mit einem Substrat aus organischen Fasern, die anhängende Hydroxylgruppen aufweisen, einem Bindemittel, das auf mindestens einen Teil der Fasern aufgebracht ist, wobei das Bindemittel einen vernetzten Polyvinylalkohol und ein hydrophobes Polymer umfasst, das aus einer hydrophoben Latexemulsion stammt, und einem im Bindemittel verteilten Pigment.

Der Erfindung liegt die Aufgabe zugrunde, die Oberfläche von aus nieder-energetischen Chemiefasern hergestellten Produkten dauerhaft mit funktionellen Gruppen auszurüsten, wobei dies im Wesentlichen mit einer bekannten und als Massenware kommerziell erhältlichen Chemikalie erreicht wird und die Oberflächenmodifizierung im industriellen Maßstab erfolgen kann. - Diese dauerhafte Funktionalisierung der Materialioberflächen erlaubt die ionoge und/oder kovalente Bindung von wasserlöslichen Chemikalien, z.B. von Farbstoffen oder biologisch aktiven Verbindungen, verbessert die Wasserbenetzbarkeit, verbessert das Wasseraufnahme- und Wasserhaltevermögen, erlaubt einen kapillaren Wassertransport etc., insgesamt vergrößert sich dadurch das Einsatzspektrum von nieder-energetischen Materialien.

Die Aufgabe wird durch ein Verfahren nach Anspruch 1 gelöst.

Silanol(at)gruppen-haltiger Polyvinylalkohol ist bekannt und im industriellen Maßstab kommerziell erhältlich, siehe hierzu das Datenblatt "Kuraray™ R-Polymers" von November 2007 der Firma Kuraray, Frankfurt am Main.

Die Formel des silanol(at)gruppen-haltigen Polyvinylalkohol ist -CH(OH)-CH₂-CH(OAc)-CH₂-CH(Si(ONa)₃-CH₂-CH(OAc)-CH₂-CH(OH)-CH₂- wobei Ac Acetyl (CH₃CO-) ist. Der Anteil an Vinylacetatgruppen beträgt laut

Datenblatt 1,5 ± 0,5 mol-%. Die Silanol(at)gruppen sind hydrolysestabil über =C-Si= -Bindungen an die Polymerkette gebunden. Die Säurekonstante pKa von Silanolgruppen liegt bei pH 4, d.h. dass bei pH <4 ungeladene Silanol- (-Si(OH)₃) Gruppen vorliegen; bei pH >4 negativ geladene Silanolat- (-Si(O⁻)₃) Gruppen vorliegen.

Zur dauerhaften Oberflächenmodifizierung werden wässrige Lösungen von Silanol(at)gruppen-haltigem Polyvinylalkohol eingesetzt. Die eingesetzte Konzentration des Polymeren richtet sich insbesondere nach dem späteren Verwendungszweck des Produkts und kann zwischen 0,001% (w/v) und 40% (w/v) variieren, in der Regel liegen die Konzentrationen zwischen 0,01% (w/v) und 10% (w/v). Das Herstellen derartiger Lösungen ist bekannt, siehe u.a. das oben erwähnte Datenblatt.

Diesen Polymer-Lösungen können weitere, die Oberflächenspannung von Wasser herabsetzende Substanzen, wie beispielsweise nieder-aliphatische Alkohole, Tenside, Netzmittel zugesetzt sein. Verbindungen, die die Viskosität der Lösung modifizieren, wie zum Beispiel pyrogene Kieselsäure oder Metallsalze, können ebenfalls Bestandteil der Lösung sein. Deren, dem Fachmann geläufige Auswahl erfolgt im Hinblick auf den späteren Verwendungszweck der oberflächen-modifizierten Materialien, der Kompatibilität mit dem gelösten modifizierten Polyvinylalkohol, den zu modifizierenden Materialtypen und Materialeigenschaften, den Kontaktierungs- und Trocknungsbedingungen.

Der pH der eingesetzten Polymer-Lösung liegt vorzugsweise im Sauren, bei 3 ± 1.

Die Temperatur der eingesetzten Polymer-Lösung kann zwischen 3°C und 95°C liegen, vorzugsweise liegt sie zwischen 15 und 65°C, ganz bevorzugt bei Raumtemperatur.

Die nieder-energetischen Materialien werden mit einer wässrigen Silanol(at)-gruppen-haltigen Polyvinylalkohol-Lösung kontaktiert. Der Begriff "Kontakieren" beschreibt hier, dass die gesamte oder bestimmte Teile der Oberfläche des nieder-energetischen Materials in die Lage versetzt wird, mit den gelösten Stoffen in der Lösung in physikalisch-chemische Wechselwirkungen zu treten und umfasst auf molekularer Ebene Begriffe wie Physisorption und Chemiesorption und auf produktionstechnischer Ebene Begriffe wie in-innigen-Kontakt-bringen, beschichten, umhüllen, lackieren, benetzen, imprägnieren, besprühen, bestreichen, (ein)tauchen, etc.. Bevorzugt werden hierfür bekannte, im industriellen Maßstab eingesetzte Hilfsmittel wie (Finishing)Bäder, Foulard, Rakel, Kiss-Roll, Auftragswalze, WEKO®-Rotorenfeuchtung etc. eingesetzt.

Die Kontaktierungsdauer der nieder-energetischen Materialien mit der Polymer-Lösung liegt im Bereich von Sekunden und mehreren Tagen und wird jeweils unter Berücksichtigung der eingesetzten Polymer-Konzentration, des späteren Einsatzzweck des Materials, dessen Eigenschaften, wie Texturierung, Kompaktheit, Flächengewicht etc. und auch ökonomischen Gesichtspunkten ermittelt und optimiert.

Nach dem die nieder-energetischen Materialien mit der Polymer-Lösung kontaktiert wurden, wird das Lösemittel, vorzugsweise Wasser, entfernt, was üblicherweise durch Trocknung der mit Polymer-Lösung benetzten Materialien erfolgt. Die Trocknungstemperaturen können zwischen 1°C und über 170°C liegen. Sie werden von Fall zu Fall, unter Berücksichtigung des späteren Einsatzzwecks und den Eigenschaften des eingesetzten Materials, festgelegt. Bevorzugt liegen sie bei Raumtemperatur, besonders bevorzugt zwischen 30 und 70°C und ganz besonders zwischen 70 und 130°C. Der Grad der Trocknung richtet sich nach dem späteren Einsatzzweck der Materialien, so dass diese eine Restfeuchte zwischen 0% und 40%, bezogen auf nicht-oberflächenmodifizierte Materialien, aufweisen können.

Überraschendes Ergebnis oben beschriebener Kontaktierung von nieder-energetischen Materialien mit wässrigen Lösungen von Silanol(at)gruppenhaltigem Polyvinylalkohol und anschließender Trocknung war, dass derart behandelte Materialien mit einem wasserlöslichen Farbstoff für Baumwolle (Handelsname simplicol®, Firma Brauns-Heitmann, Warburg) angefärbt werden konnten und dass die Färbung auch nach intensiven Waschen mit warmen Wasser nicht ausgewaschen werden konnte. Dies wird als Beweis für das dauerhafte Vorhandensein von funktionellen Gruppen, in diesem Fall von Hydroxyl- (-OH) Gruppen, auf der Materialoberfläche gewertet. Erfindungsgemäß erhält somit die inerte Oberfläche einer Chemiefaser die chemisch-reaktiven Eigenschaften von Cellulosefasern, so dass man von "künstlicher Cellulose" bzw. "künstlicher Baumwolle" sprechen kann.

Es wird davon ausgegangen, dass die oben beschriebene Kontaktierung und die anschließende Trocknung bewirkt, dass sich die mit Silanol(at)gruppen funktionalisierten Polyvinylalkohol-Moleküle mit fortschreitender Verdunstung des Lösemittel Wasser zunächst auf der Oberfläche der Chemiefaser anreichern. Im weiteren Verlauf reagieren dann die Silanol(at)gruppen (durch Kondensationsreaktion und unter Wasserabspaltung vorwiegend intermolekular) miteinander, so dass sich ein Riesenmolekül, das dann die Materialoberfläche wie eine Lackschicht fest umhüllt, ausbildet. Durch gezielte Einstellung der Konzentration der silanol(at)gruppen-haltigen Polyvinylalkohol-Lösung lässt sich erreichen, dass sich sowohl dünne, möglicherweise auch monomolekulare Schichten auf der Oberfläche aufbringen lassen, aber auch dicke Schichten, die die einzelnen Fasern fest miteinander verbinden oder aber auch Maschen- / Gewebelücken verschließen.

An die derartig fest auf der Oberfläche gebundenen funktionellen Hydroxy-(-OH) Gruppen können in einem weiteren Schritt wasserlösliche Substanzen über Ester- oder Acetal- oder Urethan- oder Etherbindungen kovalent gebunden werden. Die Veresterung kann mit anorganischen oder organischen Säuren erfolgen. Beispielsweise kann man die -OH-Gruppen mit Di-, Tri- oder Polycarbonsäuren verestern, so dass die Materialoberfläche in wässriger Lösung negativ, anionisch geladen ist. Durch Veresterung mit Zwitterionen, z.B. Aminosäuren, bekommt die Oberfläche eine kationische, positive Ladung, die für weitere Reaktionen genutzt werden kann, siehe Beispiel 3. Aldehydgruppen-haltige Verbindungen, wie zum Beispiel Glycerinaldehyd, lassen sich säure-katalysiert unter Ausbildung einer Acetalbindung an die -OH-Gruppen addieren, in diesem Falle erhöht sich die Anzahl an -OH-Gruppen auf der Materialoberfläche. Doppelbindungs-haltige Verbindungen, wie z.B. 5-Pentensäure, lassen sich peroxidisch-katalysiert unter Ausbildung von hydrolysestabilen Etherbindungen an die -OH-Gruppen addieren. In diesem Fall bekommt die Oberfläche, bei pH >5, durch die -COO⁻ -Gruppen eine negative, anionische Ladung. - Diese zusätzlichen Oberflächenmodifikationen verschieben das Einsatzspektrum von derart oberflächen-modifizierten nieder-energetischen Materialien von textilen Anwendungsbereichen hin zu Anwendungsbereichen in Medizin und Biotechnologie.

Die gemäß dem Verfahren oberflächen-modfizierten aus nieder-energetischen Chemiefasern hergestellten Produkte werden als Faden- und/oder Fasermaterial für textile Flächengebilde, als textile Flächengebilde, als textile Flächengebilde, die mit wasserlöslichen Farbstoffen färbbar sind, als geotextile Flächengebilde, als Filtermaterialien, als Wundauflagen, als Hygieneartikel, als Substrate zur Immobilisierung biologisch aktiver Substanzen, als Zellkultursubstrate, als Substrate, die Metallisierbar sind, als sinkfähige Angelschnur etc. eingesetzt.

### Beispiel 1:

Als Silanol(at)gruppen-haltiger Polyvinylalkohol wird R-Polymer R-1130™ der Firma Kuraray, Frankfurt eingesetzt.

Lösung: Zur Oberflächenmodifizierung nieder-energetischer Materialien wird eine 1%ige (w/v) wässrige R-1130™-Lösung verwendet, die wie folgt hergestellt wurde: bei Raumtemperatur werden 10 g R-Polymer™ in 1.000 ml de-ionisiertes Wasser vorgelegt und dann unter ständigen Rühren auf ca. 90°C erwärmt bis das Polymer vollständig gelöst hat. Der pH wird mit verdünnter Essigsäure auf 3 eingestellt.

Material: Faserverbundstoff: Spinnvliese aus Polypropylen mit jeweils 10, 20 und 30 g/m² Flächengewicht der Firma Fiberweb Corovin, Peine.

Kontaktierung: ca. fünf DIN A4 große Vliese werden übereinander in eine passende Schale gelegt und ca.100 ml o.g. R-Polymer-Lösung wird auf die Vliese gegossen. Aufgrund der Hydrophobie der Vliese dringt die Lösung nicht in die Vliese ein, sondern perlt ab. Eine vollständige, luftblasenfreie Benetzung mit der Polymer-Lösung ist nur mit Hilfe eines Rollenquetschers möglich (dieser stellt den Foulard in einer industriellen Anwendung dar).

Trocknung: die Vliese werden tropfnass auf eine Wäscheleine gehängt und bei Raumtemperatur getrocknet.

Ergebnis: im Vergleich zu unbehandelten Vliesen lassen sich alle Vliese vollständig, auch nach mehrmaligem Trocknen, immer wieder mit Wasser benetzen. Getrocknete Vliese fassen sich im unteren Bereich deutlich steifer an als im oberen Bereich, wo sie mit Wäscheklammern auf der Leine fixiert waren. Dieser Unterschied im Griff ist bei dem 35 g/m² Vlies deutlicher als bei den anderen und bleibt auch nach mehrmaligem Waschen mit Wasser erhalten.

Es zeigt sich eine deutliche, nicht auswaschbare Färbung mit simplicol® (ein Farbstoff zur Färbung von Baumwolle der Firma Brauns-Heitmann, Warburg) - unbehandelte Kontrollen binden keinen Farbstoff.

### Beispiel 2:

Lösung: wie bei Beispiel 1 mit zusätzlich 2% (v/v) 2-Propanol
Material: Faserverbundstoff: DIN A4 große Nadelvliese aus Polypropylen, Sawatex® 11311Di52 25g/m² der Firma Sandler, Schwarzenbach.
Kontaktierung: manuell, ähnlich Beispiel 1, aber keine Verwendung eines Rollenquetschers, da die Vliese aufgrund des zugesetzten 2-Propanol sofort vollständig benetzt wurden.
Trocknung: wie Beispiel 1.

Ergebnis (WSP Prüfmethode, jeweils im Vergleich zur unbehandelten Kontrolle):
* maximale Wasseraufnahme: 1.200 vs. 485
* Durchdringzeit, 3 Zyklen (repeated strike through): stets <4 sec vs. >40 sec Deutliche Färbung mit simplicol®, fehlende Farbbindung bei unbehandelter Kontrolle.

### Beispiel 3:

Lösung: wie Beispiel 1 mit Zusatz von 0,25% (v/v) Silastan RN Neu® (ein Netzmittel der Firma Schill und Seilacher, Böblingen).
Material: Faserverbundstoff: ein auf der Technikumsanlage der Firma Trützschler, Egelsbach durch Wasserstrahlvernetzung von Stapelfasern aus Polypropylen hergestelltes Vlies mit einem Flächengewicht von 45 g/m² und 900 mm Breite. Die Herstellung des Vlieses sowie dessen Kontaktierung mit Polymer-Lösung und Trocknung erfolgte bei unterschiedlichen, zwischen 30 und 100 m/min liegenden, Laufgeschwindigkeiten der Anlage.
Kontaktierung: in-line, direkt nach der Vliesherstellung mittels Sprühvorrichtung der Firma Weko Biel AG, CH-Biel.
Trocknung: in-line, direkt nach der Kontaktierung mittels industriell eingesetztem Trockenofen bei einer Trocknungstemperatur von 110°C.
Ergebnis: bei Kontakt mit Wasser saugt sich das Vlies sofort voll und versinkt im Wasser. Durch mehrmaliges Waschen mit Wasser und anschließender Trocknung verlangsamt sich die Wasserabsorption, bleibt aber erhalten.

Die gebundenen Hydroxyl- (-OH) Gruppen lassen sich mittels simplicol®-Färbung stets nachweisen, wobei Vliese, die mit geringerer Laufgeschwindigkeit "gefahren" wurden, intensiver gefärbt sind.

Mit dem kationischen Farbstoff Pyronin G™ kann das Vlies leicht angefärbt werden. Dies wird darauf zurückgeführt, dass Silanolgruppen, die an der Kondensation nicht teilgenommen haben, nunmehr, bei der Färbung bei pH 7, anionische Silanolat- (-Si(O₃⁻)) Gruppen sind, die den kationischen Farbstoff ionogen binden.

Die Hydroxyl- (-OH) Gruppen wurden mit Betain verestert (= Kontaktierung der Vliese über Nacht mit einer ca. 2,5%ige (w/v) wässrigen Betain-Lösung, pH 3). An die nicht gebundenen kationischen Trimethylammonium-Gruppen des Betain binden dann aus wässriger Phase heraus anionische Verbindungen, wie z.B. Kupfer(II)-phthalocyanin-tetrasulfonsäure Tetranatriumsalz.

### Beispiel 4:

Lösung: wie bei Beispiel 3.
Material: im Wesentlichen 1-dimensionale Chemiefaser: 722 Filamente aus Polypropylen mit 2,7 dtex und im km-Bereich liegender Länge, die in der Pilotanlage der Firma FiberVisions, DK-Varde, durch Schmelzspinnen hergestellt wurden
Kontaktierung: direkt nach Austritt aus der Spinndüse werden die 722 Filamente kontinuierlich mit Produktionsgeschwindigkeit durch ein Bad mit der Lösung geführt und anschließend zu einem "small tow" vereinigt und aufgewickelt
Trocknung: als "small tow" aufgewickelt bei Raumtemperatur, Trocknungszeit: mehrere Tage
Ergebnis: Positive Färbung mit simplicol®.
Deutlicher Kapillareffekt: ein in (gefärbtes) Wasser eingetauchtes Ende eines "small tow" zieht innerhalb von 24 Stunden das Wasser über eine Strecke von ca. 300 mm senkrecht nach oben.

### Beispiel 5:

Lösung: wie bei Beispiel 2.
Material: Fadenverbundstoff: T-Shirt aus Polyethylenterephthalat
Kontaktierung: manuelles, luftblasenfreies Einwalken der Lösung
Trocknung: manuelles Auswrinken und auf Wäscheleine bei Raumtemperatur
Ergebnis: im Vergleich zur unbehandelten Kontrolle trocknet das T-Shirt auch nach mehrmaligem Waschen wesentlich langsamer

## Patentansprüche

1. Verfahren zur Oberflächenmodifizierung von aus nieder-energetischen Chemiefasern hergestellten textilen Produkten, die eine freie Oberflächenenergie unter 40 mN/m und/oder einen Kontaktwinkel mit Wasser von größer als 45° aufweisen, **dadurch gekennzeichnet, dass** die Produkte mit einer wässrigen silanol(at)gruppen-haltigen Polyvinylalkohol-Lösung kontaktiert und das Lösemittel wieder entfernt wird, wobei der pH der wässrigen silanol(at)gruppen-haltigen Polyvinylalkohol-Lösung zwischen 2 und 5 liegt, wobei die Lösung aus Wasser und silanol(at)gruppen-haltigen Polyvinylalkohol, sowie gegebenenfalls weiteren Substanzen, die die Oberflächenspannung und/oder die Viskosität der Lösung beeinflussen, besteht, und wobei die textilen Produkte:
(i) aus der Gruppe bestehend aus Filamenten, Mono-filamenten, Multifilamenten, Fasern, Fäden, Garnen, Zwirnen und Rovings ausgewählt sind; oder
(ii) als Fadenverbundstoffe aus der Gruppe bestehend aus Geweben, Gewirken, Fleece, Gestricken, Netzen und Geflechten vorliegen; oder
(iii) als Faserverbundstoff aus der Gruppe bestehend aus Vliesen, Filzen und Watte vorliegen; oder
(iv) als Kombinationen aus Faden- und Faserverbundstoff in Form von Flächenschichtstoffen vorliegen.

2. Verfahren gemäß Anspruch 1, **dadurch gekennzeichnet, dass** der gelöste silanol(at)gruppen-haltige Polyvinylalkohol die Formel -CH(OH)-CH₂-CH(OAc)-CH₂-CH(Si(ONa)₃-CH₂-CH(OAc)-CH₂-CH(OH)-CH₂- hat, wobei Ac Acetyl (CH₃CO-) ist.

3. Verfahren gemäß Anspruch 1, **dadurch gekennzeichnet, dass** die Konzentration der silanol(at)gruppen-haltigen Polyvinylalkohol-Lösung im Bereich zwischen 0,001% (w/v) und 40% (w/v) liegt.

4. Verfahren gemäß Anspruch 1, **dadurch gekennzeichnet, dass** der pH der wässrigen silanol(at)gruppen-haltigen Polyvinylalkohol-Lösung zwischen 2 und 4 liegt.

5. Verfahren gemäß Anspruch 1, **dadurch gekennzeichnet, dass** die gesamte oder Teile der Oberfläche von aus nieder-energetischen Chemiefasern hergestellten Produkte mit der wässrigen silanol(at)gruppen-haltigen Polyvinylalkohol-Lösung kontaktiert wird.

6. Verfahren gemäß Anspruch 5, **dadurch gekennzeichnet, dass** die Kontaktierung manuell erfolgt.

7. Verfahren gemäß Anspruch 5, **dadurch gekennzeichnet, dass** die Kontaktierung unter Zuhilfenahme bekannter maschineller Hilfsmittel in-line und bei Produktionsgeschwindigkeit erfolgt.

8. Verfahren gemäß Anspruch 1, **dadurch gekennzeichnet, dass** die Entfernung des Lösemittels der mit der wässrigen silanol(at)gruppen-haltigen Polyvinylalkohol-Lösung kontaktierten Produkte durch Trocknung geschieht.

9. Verfahren gemäß Anspruch 8, **dadurch gekennzeichnet, dass** die Trocknung bei Temperaturen zwischen 1°C und 130°C erfolgt.

10. Verfahren gemäß Anspruch 8, **dadurch gekennzeichnet, dass** die Trocknung bei Raumtemperatur auf einer (Wäsche) Leine erfolgt.

11. Verfahren gemäß Anspruch 8, **dadurch gekennzeichnet, dass** die Trocknung unter Zuhilfenahme bekannter maschineller Hilfsmittel in-line und bei Produktionsgeschwindigkeit erfolgt.

12. Verfahren gemäß Anspruch 8, **dadurch gekennzeichnet, dass** die Restfeuchte nach Trocknung zwischen 0% und 40% beträgt.

13. Aus nieder-energetischen Chemiefasern hergestellte textile Produkte, **dadurch gekennzeichnet, dass** die Produkte mit einer silanol(at)gruppen-haltigen Polyvinylalkohol-Lösung gemäß Anspruch 1 kontaktiert und das Lösemittel wieder entfernt wurde.

14. Produkte gemäß Anspruch 13, **dadurch gekennzeichnet, dass** sie als Substrate zur kovalenten Bindung von wasserlöslichen Substanzen über Ester-, Urethan-, Acetal- oder Etherbindungen dienen.

15. Verwendung der aus nieder-energetischen Chemiefasern hergestellten und gemäß dem Verfahren nach einem der Ansprüche 1 bis 12 oberflächen-modifizierten Produkte als
- sinkfähige Angelschnur
- Transportmittel für (Grund)Wasser, z.B. für kapillar-aktive künstliche Wurzeln
- Ausgangsmaterial für textile Flächengebilde
- textiles Flächengebilde
- geotextiles Flächengebilde
- Filtermaterial für wässrige Medien
- blut und sekret-aufsaugende Wundauflage
- flüssigkeit-aufnehmende und -durchleitende Hygieneartikel
- Substrat zur Immobilisierung biologisch aktiver Substanzen
- Zellkultursubstrat
- Substrat für (stromlose) Metallisierung.

## Claims

1. A method for the surface modification of textile products which are made from low-energy synthetic fibres and have a free surface energy of less than 40 mN/m and/or a contact angle with water of greater than 45°, **characterized in that** the products are contacted with an aqueous polyvinyl alcohol solution containing silanol(ate) groups and the solvent is removed again, the pH of the aqueous polyvinyl alcohol solution containing silanol(ate) groups being between 2 and 5, the solution consisting of water and polyvinyl alcohol containing silanol(ate) groups and, optionally, of further substances that influence the surface tension and/or the viscosity of the solution, and wherein the textile products:
(i) are selected from the group consisting of filaments, monofilaments, multifilaments, fibres, threads, yarns, strings and rovings; or
(ii) are present as filament composite materials from the group consisting of woven fabrics, warp knitted fabrics, fleece, weft knitted fabrics, nets and braids; or
(iii) are present as a fibre composite material from the group consisting of nonwovens, felts and wadding; or
(iv) are present as combinations of filament and fibre composite materials in the form of textile multilayer composites.

2. The method according to claim 1, **characterized in that** the dissolved polyvinyl alcohol containing silanol(ate) groups is represented by the formula -CH(OH)-CH₂-CH(OAc)-CH₂-CH(Si(ONa)₃-CH₂-CH(OAc)-CH₂-CH(OH)-CH₂-, wherein Ac is acetyl (CH3CO-).

3. The method according to claim 1, **characterized in that** the concentration of the polyvinyl alcohol solution containing silanol(ate) groups ranges from 0.001% (w/v) to 40% (w/v).

4. The method according to claim 1, **characterized in that** the pH of the aqueous polyvinyl alcohol solution containing silanol(ate) groups is between 2 and 4.

5. The method according to claim 1, **characterized in that** all or parts of the surface of products made from low-energy synthetic fibres is/are contacted with the aqueous polyvinyl alcohol solution containing silanol(ate) groups.

6. The method according to claim 5, **characterized in that** the contacting is effected manually.

7. The method according to claim 5, **characterized in that** the contacting is effected with the aid of known mechanical aids, in-line and at production speed.

8. The method according to claim 1, **characterized in that** the solvent is removed from the products contacted with the aqueous polyvinyl alcohol solution containing silanol(ate) groups by drying.

9. The method according to claim 8, **characterized in that** the drying takes place at temperatures between 1°C and 130°C.

10. The method according to claim 8, **characterized in that** the drying is carried out at room temperature on a (washing) line.

11. The method according to claim 8, **characterized in that** the drying is effected with the aid of known mechanical aids, in-line and at production speed.

12. The method according to claim 8, **characterized in that** the residual moisture after drying is between 0% and 40%.

13. Textile products made from low-energy synthetic fibres and **characterized in that** the products were contacted with a polyvinyl alcohol solution containing silanol(ate) groups according to claim 1 and the solvent was removed again.

14. The products according to claim 13, **characterized in that** they are used as substrates for the covalent bonding of water-soluble substances via ester, urethane, acetal or ether bonds.

15. Use of the products made from low-energy synthetic fibres and surface-modified in accordance with the method according to any of claims 1 to 12 as
- sinkable fishing line
- means of conveyance for (ground)water, e.g. for capillary active artificial roots
- starting material for textile webs
- textile web
- geotextile web
- filter material for aqueous media
- blood- and body fluid-absorbing wound dressing
- fluid-absorbent and -permeable sanitary articles
- substrate for the immobilisation of biologically active substances
- cell cultivation substrate
- substrate for (non-electric) metallisation.

## Revendications

1. Procédé de modification de surface de produits textiles qui sont fabriqués à partir de fibres synthétiques à faible énergie et qui présentent une énergie superficielle libre inférieure à 40 mN/m et/ou un angle de contact avec de l'eau supérieur à 45°, **caractérisé en ce que** les produits sont amenés en contact avec une solution aqueuse d'alcool polyvinylique contenant des groupes de silanol(ate) et **en ce que** le solvant est de nouveau enlevé, le pH de la solution aqueuse d'alcool polyvinylique contenant des groupes de silanol(ate) étant compris entre 2 et 5, la solution étant composée d'eau et d'alcool polyvinylique contenant des groupes de silanol(ate) et, le cas échéant, d'autres substances influençant la tension superficielle et/ou la viscosité de la solution, et dans lequel les produits textiles :
(i) sont choisis dans le groupe constitué de filaments, de monofilaments, de multifilaments, de fibres, de brins, de fils, de fils retordus et de mèches ; ou
(ii) sont présents sous forme de matières composites de fils du groupe constitué de tissus, de maillages, de polaires, de tricotages, de filets et de tresses ; ou
(iii) sont présents sous forme de matière composite de fibres du groupe constitué de non-tissés, de feutres et de ouate ; ou
(iv) sont présents en tant que combinaisons de matières composites de fils et de fibres sous forme de stratifiés de surface.

2. Procédé selon la revendication 1, **caractérisé en ce que** l'alcool polyvinylique contentant des groupes de silanol(ate) dissous a la formule -CH(OH)-CH₂-CH(OAc)-CH₂-CH(Si(ONa)₃-CH₂-CH(OAc)-CH₂-CH(OH)-CH₂-, Ac étant de l'acétyle (CH3CO-).

3. Procédé selon la revendication 1, **caractérisé en ce que** la concentration de la solution d'alcool polyvinylique contenant des groupes de silanol(ate) est dans la plage comprise entre 0,001% (w/v) et 40% (w/v).

4. Procédé selon la revendication 1, **caractérisé en ce que** le pH de la solution aqueuse d'alcool polyvinylique contenant des groupes de silanol(ate) est compris entre 2 et 4.

5. Procédé selon la revendication 1, **caractérisé en ce que** l'ensemble ou des parties de la surface de produits fabriqués à partir de fibres synthétiques à faible énergie est/sont amené(es) en contact avec la solution aqueuse d'alcool polyvinylique contenant des groupes de silanol(ate).

6. Procédé selon la revendication 5, **caractérisé en ce que** la mise en contact est réalisée manuellement.

7. Procédé selon la revendication 5, **caractérisé en ce que** la mise en contact est réalisée à l'aide de moyens mécaniques connus, en ligne et à la vitesse de production.

8. Procédé selon la revendication 1, **caractérisé en ce que** le retrait du solvant des produits amenés en contact avec la solution aqueuse d'alcool polyvinylique contenant des groupes de silanol(ate) est réalisé par séchage.

9. Procédé selon la revendication 8, **caractérisé en ce que** le séchage est réalisé à des températures entre 1°C et 130°C.

10. Procédé selon la revendication 8, **caractérisé en ce que** le séchage est réalisé à température ambiante sur une corde (à linge).

11. Procédé selon la revendication 8, **caractérisé en ce que** le séchage est réalisé à l'aide de moyens mécaniques connus, en ligne et à la vitesse de production.

12. Procédé selon la revendication 8, **caractérisé en ce que** l'humidité résiduelle après le séchage est comprise entre 0% et 40%.

13. Produits textiles fabriqués à partir de fibres synthétiques à faible énergie, **caractérisés en ce que** les produits ont été amenés en contact avec une solution d'alcool polyvinylique contenant des groupes de silanol(ate) selon la revendication 1 et **en ce que** le solvant a été de nouveau enlevé.

14. Produits selon la revendication 13, **caractérisés en ce qu'**ils servent de substrats pour la liaison covalente de substances solubles dans l'eau par l'intermédiaire de liaisons d'ester, d'uréthane, d'acétal ou d'éther.

15. Utilisation des produits fabriqués à partir de fibres synthétiques à faible énergie et à surface modifiée selon le procédé selon l'une des revendications 1 à 12 en tant que
- fil à pêche non flottant
- moyen de transport pour de l'eau (souterraine), par exemple pour des racines artificielles à activité capillaire
- matière de base pour tissus textiles
- tissu textile
- tissu géotextile
- matière de filtrage pour milieu aqueux
- pansement pour plaies à absorption de sang et de sécrétion
- article d'hygiène absorbant les liquides et perméable aux liquides
- substrat pour l'immobilisation de substances biologiquement actives
- substrat de culture de cellules
- substrat pour une métallisation (sans courant).
